# EUROPEAN PATENT SPECIFICATION

(11) **EP 1 699 764 B1**
(45) Date of publication and mention of the grant of the patent: **06.06.2007**
(21) Application number: 04804148.7
(22) Date of filing: 21.12.2004
(51) Int. Cl.: C07D 223/16

(54) **BIS-DICARBOXYLIC ACID SALTS OF BENAZEPRIL AND PREPARATION OF BENAZEPRIL VIA THESE SALTS**
BIS-DICARBONSÄURESALZE VON BENAZEPRIL UND HERSTELLUNG VON BENAZEPRIL ÜBER DIESE SALZE
SELS D'ACIDE BIS-DICARBOXYLIQUE DE BENAZEPRIL ET PREPARATION DE BENAZEPRIL A PARTIR DE CES SELS

(30) Priority: 22.12.2003 US 531746 P
(43) Date of publication of application: 13.09.2006
(73) Proprietor: Meda AB, 170 09 Solna (SE)
(72) Inventor: GRIMLER, Dominique, F-68560 Hirsingue (FR); SEDELMEIER, Gottfried, 79227 Schallstadt (DE)
(74) Representative: Endler, Gabriele
(86) International application number: PCT/EP2004/014552
(87) International publication number: WO 2005/066134

(56) References cited:
- US-A- 4 575 503

## Description

The present invention is directed to novel salts of esters of benazepril, which are convertible to benazepril hydrochloride.

Benazepril hydrochloride is marketed as an angiotensin-converting enzyme inhibitor which is useful for the treatment of hypertension in man.

Benazepril is 3-[[1-(ethoxy-carbonyl)-3-phenyl-(*1S*)-propyl]amino]-2,3,4,5-tetrahydro-2-oxo-*1H*-1-(*3S*)-benzazepine-1-acetic acid; its structural formula (I) is 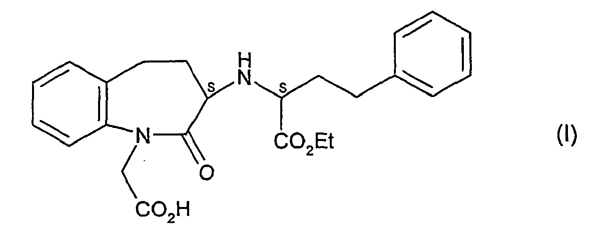 in which S indicates the (S)-configuration at the asymmetric carbon atoms.

Benazepril hydrochloride is the active ingredient of Lotensin^{®} tablets and Lotensin HCT^{®} tablets, and is one of the active ingredients of Lotrel^{®} capsules, all marketed by Novartis Pharmaceuticals Corporation, East Hanover, NJ, USA, for the treatment of hypertension.

As seen above, benazepril hydrochloride is the (*S*,*S*)-diastereomer which is one of four possible diastereomers, namely the (*S,S*)*,* (*R,R*)*,* (*R,S*) and (*S,R*) diastereomers.

The preparation of benazepril and benazepril hydrochloride has been described in U.S. Patent Nos. 4,410,520; 4,785,089; 4,918,187 and 5,098,841.

An object of the present invention is to provide benazepril and pharmaceutically acceptable salts thereof of high purity, in particular, of high enantiomeric purity.

The last step in the preparation of benazepril hydrochloride is the selective removal of the protecting group R in a diester of formula (II) 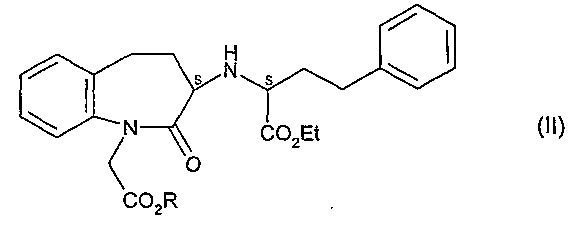 wherein R represents a selectively removable protecting group, e.g., optionally substituted benzyl or benzhydryl (substituted, e.g., by nitro), *t*-butyl, allyl, tetrahydrofuranyl or tetrahydropyranyl.

Such carboxylic acid ester protecting groups are well-known in the art, e.g., as described in Greene, *Protective Groups in Organic Synthesis,* John Wiley and Sons and, e.g., as used in penicillin and cephalosporin synthesis.

Allyl esters (the protecting group R being allyl) are described, e.g., in *Peptide Protein* Res, Vol. 26, pp. 493-497 (1985). The allyl ester can be prepared, e.g., by treatment of the carboxylic acid with allyl bromide in the presence of, e.g., cesium carbonate.

Diesters of formula (II) are prepared as illustrated, e.g., in U.S. Patent No. 4,785,089, which is incorporated here by reference. The reaction sequence is as follows: 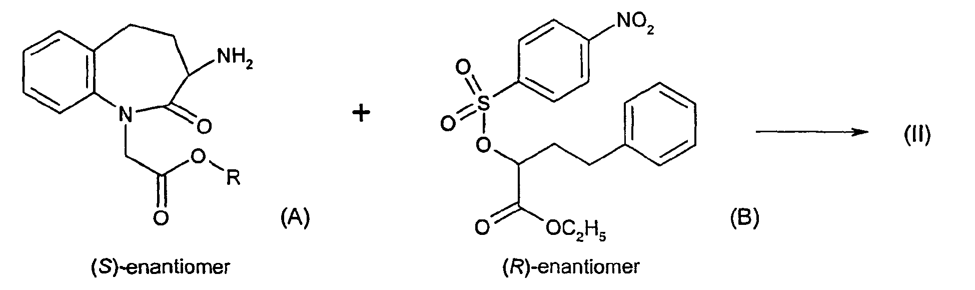 R represents a selectively removable protecting group.

The enantiomeric purity of (A), (B) and (II) starting materials is about 95-99%.

It has now been discovered that the basic diesters of formula (II) unexpectedly form crystalline bis-dicarboxylic acid addition salts with dicarboxylic acids, that is salts in which the molecular ratio of a compound of formula (II) and the dicarboxylic acid is 1:2. A particular embodiment relates to the salts of formula (III) 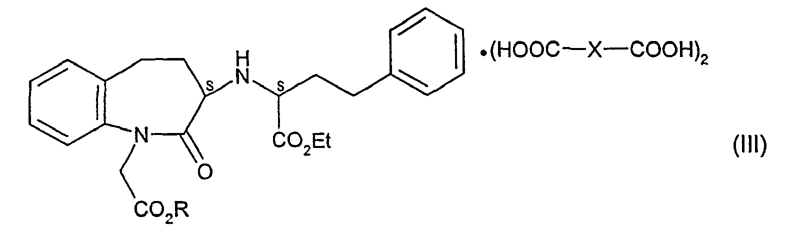 in which
X represents a direct bond, lower alkylene, arylene or mono- or *bis*-hydroxy-lower alkylene; and
R has the meaning as defined above.
Lower alkylene represents straight-chain or branched alkylene of 1-7 carbon atoms.
Arylene represents, e.g., phenylene, advantageously para-phenylene.
Representative dicarboxylic acids of the formula (IV)

   HOOC-X-COOH (IV)
are oxalic acid, *D*- or *L*-tartaric acid, optionally substituted (malonic acid, glutaric acid or succinic acid), *D-* or *L-*malic acid, *D*- or *L*-citramalic acid, terephthalic acid, aspartic acid and the like.

Optionally substituted (malonic acid, glutaric acid or succinic acid) refers to such being unsubstituted or substituted by, e.g., lower alkyl, and lower alkyl represents straight chain or branched alkyl of 1-7 carbon atoms.

Particular embodiments of salts of formula (III) are, e.g., salts of formula (III) wherein R is *t*-butyl; and the dicarboxlyic acid of the formula (IV)

HOOC-X-COOH (IV)

is oxalic acid, tartaric acid or isopropylmalonic acid.

The salts are typically prepared by treating one mole of a compound of formula (II) with 1-2 mole equivalents, preferably 2 mole equivalents, of the dicarboxylic acid in an organic solvent, at a temperature ranging from about room temperature to the reflux temperature of the solvent and, if required, purified by recrystallization using an appropriate solvent or mixture of solvents.

The new highly enantiomerically pure diester salts of formula (III) are useful as intermediates for the preparation of benazepril and salts thereof of high enantiomeric purity. The enantiomeric purity of the final product (benazepril) and salts thereof is about 99-100%, preferably about 99.6-100%, advantageously 99.8-100%.

The diester dicarboxylic acid salts of formula (III) are selectively converted to benazepril or a salt thereof by selective removal of the protecting group R using methodology well-known in the art.

For example, the *t*-butyl ester or the benzhydryl ester group is cleaved by treatment with an anhydrous acid, e.g., by solvolysis with HCl gas in an organic solvent, such as ethyl acetate to obtain benazepril hydrochloride.

Benzyl esters are selectively converted to the acid by, e.g., catalytic hydrogenolysis, e.g., in the presence of Pd on charcoal.

Benzhydryl (diphenylmethyl) esters are cleaved under anhydrous acidic conditions similarly to *t*-butyl esters.

Allyl esters can be converted to the acid using a rhodium(I) or palladium(0) catalyst, e.g., tris(triphenylphosphine) rhodium(l) chloride or tetrakis(triphenylphosphine) palladium (0).

If the compound of formula (I) (the free base) is obtained, such can be converted to a pharmaceutically acceptable salt thereof, e.g., to benazepril hydrochloride using methodology known in the art.

A further aspect of the invention relates to a process for the preparation of benazepril, the structural formula (I) of which is 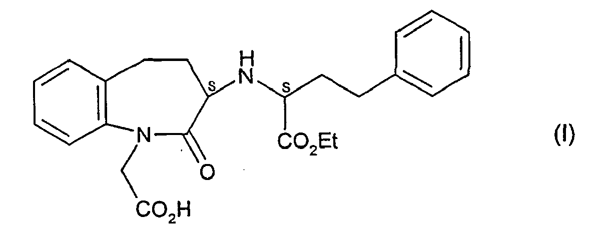 in which (S) denotes the (S)-configuration, or a pharmaceutically acceptable salt thereof, which comprises the following steps:
(a) purifying a diester of the formula (II) 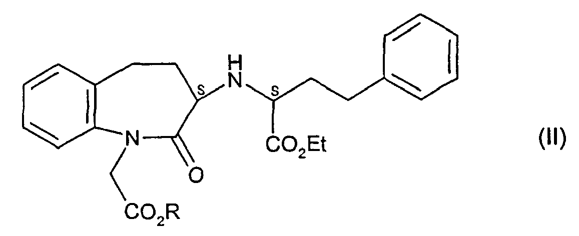 wherein
   R represents a selectively removable protecting group; and
   (S) denotes the (S)-configuration of the asymmetric carbon atoms;
   by treatment with a dicarboxylic acid to obtain a crystalline *bis*-dicarboxylic acid salt of formula (III) 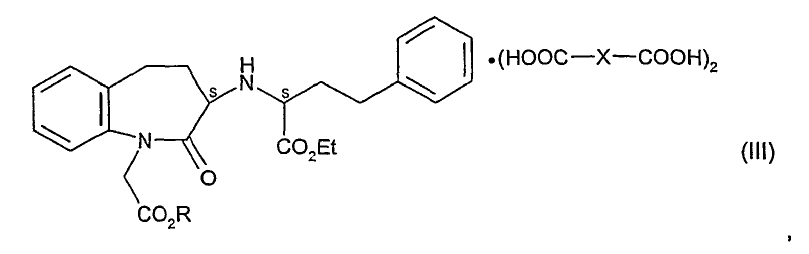 said salt being of high enantiomeric purity;
(b) converting said salt to the corresponding free base; and
(c) converting said diester free base to benazepril or a pharmaceutically acceptable salt thereof by selective removal of the R protecting group; and
(d) if required, converting the resulting benazepril free base to a pharmaceutically acceptable salt thereof.

Further embodiments thereof relate to the above process:
(a) wherein R is optionally substituted benzyl, optionally substituted benzhydryl, *t*-butyl or allyl;
(b) wherein the dicarboxylic acid is a compound of the formula (IV)

   HOOC-X-COOH (IV)

   wherein X represents a direct bond, lower alkylene, arylene or mono- or bis-hydroxy-lower alkylene;
(c) wherein the dicarboxylic acid is oxalic acid, *D*- or *L*-tartaric acid, optionally substituted malonic acid, e.g., isopropylmalonic acid, optionally substituted succinic acid, *D*- or *L*-malic acid, *D*- or *L*-citramalic acid, optionally substituted glutaric acid, or terephthalic acid;
(d) wherein the dicarboxylic acid is *D*- or *L*-tartaric acid, oxalic acid or isopropylmalonic acid; and
(e) wherein R is *t*-butyl and wherein Step (c) is carried out with HCl in an inert anhydrous organic solvent to obtain benazepril hydrochloride.

For the salt formation in Step (a) above, one mole of the compound of formula (II) is typically treated with 1 to 2 mole equivalents of the dicarboxylic acid, preferably 2 mole equivalents.

The salt formation is carried out at a temperature ranging from room temperature to the reflux temperature of the solvent.

Preferred solvents are alcohols, such as ethanol or isopropanol; esters, such as ethyl acetate; or mixtures of solvents to crystallize the product, e.g., isopropyl alcohol and heptane.

The following examples are intended to illustrate the invention and are not to be construed as limitations thereon. Temperatures are given in degrees Centigrade (°C).

The structure and composition of the products is confirmed by standard analytical methods, e.g., microanalysis and spectroscopic methods, such as IR and NMR. The abbreviations used are conventional in the art.

### Example 1

### 3-[[1-(t-Butoxy-carbonyl)-3-phenyl-(1S)-propyl]amino]-2,3,4,5-tetrahydro-2-oxo-1H-1-(3S)-benzazepine-1-acetic acid ethyl ester bis(D)-tartrate salt

135 mg (0.9 mmol) of *D-(-)*-tartaric acid are dissolved in 1.5 mL of isopropyl alcohol at reflux temperature. A solution of 200 mg (0.42 mmol) of 3-[[1-(t butoxy-carbonyl)-3-phenyl-(1S)-propyl]amino]-2,3,4,5-tetrahydro-2-oxo-1H-1-(3S)-benzazepine-1-acetic acid ethyl ester in 5 mL of heptane is added to the solution of *D-(-)*-tartaric acid. The solution is allowed to cool down to room temperature. After 30 minutes stirring at 20-22°C, the suspension is cooled down to 0°C and allowed to stand at this temperature for 48 hours. After filtration, the filter cake is washed with cold heptane and dried under vacuum at 40°C to obtain the 3-[[1-(t butoxy-carbonyl)-3-phenyl-(1S)-propyl]amino]-2,3,4,5-tetrahydro-2-oxo-1H-1-(3S)-benzazepine-1-acetic acid ethyl ester bis(D)-tartrate salt, m.p. 100-102°C. Elemental analysis: calculated for C₃₆H₄₈N₂O₁₇: C 55.38%, H 6.20%, O 34.84%. Found C: 55.14%, H 6.16%, N 3.54%, O 35.06%.

¹H NMR, 400 MHz, d₆-DMSO): 4.62 ppm (s, 4H, CHOH), 1.00 ppm (t, 3H, CH₂CH₃).

### Example 2

### 3-[[1-(t-Butoxy-carbonyl)-3-phenyl-(1S)-propyl]amino]-2,3,4,5-tetrahydro-2-oxo-1H-1-(3S)-benzazepine-1-acetic acid ethyl ester bis-oxalate salt

40.5 mg (0.45 mmol) of oxalic acid and 200 mg (0.42 mmol) of 3-[[1-(*t*-butoxycarbonyl)-3-phenyl-(*1S*)-propyl]amino]-2,3,4,5-tetrahydro-2-oxo-*1H*-1-(*3S*)-benzazepine-1-acetic acid ethyl ester are dissolved in 1.5 mL of ethyl acetate at reflux temperature. The solution is allowed to cool down to room temperature under stirring. During 24 hours, the product precipitates and the crystalline suspension is further cooled down to 0°C and allowed to stand at this temperature for 48 hours. After filtration, the filter cake is washed with cold heptane and dried under vacuum at 40°C to obtain the 3-[[1-(*t*-butoxy-carbonyl)-3-phenyl-(*1S*)-propyl]amino]-2,3,4,5-tetrahydro-2-oxo-*1H*-1-(*3S*)-benzazepine-1-acetic acid ethyl ester *bis*-oxalate salt, m.p. 111-112°C. Elemental analysis: calculated for C₃₂H₄₀N₂O₁₃: C 58.18, H 6.10, N 4.24, O 31.39. Found: C 58.43%, H 5.66%, N 4.25%, O 31.39%.

### Example 3

### 3-[[1-(t-Butoxy-carbonyl)-3-phenyl-(1S)-propyl]amino]-2,3,4,5-tetrahydro-2-oxo-1H-1-(3S)-benzazepine-1-acetic acid ethyl ester bis-isopropylmalonate salt

65.8 mg (0.45 mmol) of isopropylmalonic acid and 200 mg (0.42 mmol) of 3-[[1-(*t-*butoxy-carbonyl)-3-phenyl-(*1S*)-propyl]amino]-2,3,4,5-tetrahydro-2-oxo-1*H*-1-(*3S*)-benzazepine-1-acetic acid ethyl ester are dissolved in 1.5 mL of ethyl acetate at reflux temperature. The solution is allowed to cool down to room temperature. During 48-hours, the product precipitates and the suspension is cooled down to 0°C and allowed to stand at this temperature for 48 hours. After filtration, the filter cake is washed with cold heptane and dried under vacuum at 40°C to obtain the 3-[[1-(*t*-butoxy-carbonyl)-3-phenyl-(*1S*)-propyl]amino]-2,3,4,5-tetrahydro-2-oxo-*1H*-1-(*3S*)-benzazepine-1-acetic acid ethyl ester *bis-*isopropylmalonate salt, m.p. 100°C (decomposition).

¹H NMR, 400 MHz (d₆-DMSO): 0.88 ppm (d, 12H, CH(CH₃)₂, 1.02 ppm (t, 3H, CH₂(CH₃).

### Example 4

### Benazepril hydrochloride

15.6 g (20 mmol) of the *bis-D*-tartrate salt of Example 1 is suspended in 200 mL of EtOAc. To the suspension of the salt is added a 10% aqueous solution of potassium carbonate (ca. 120 mL) at room temperature under vigorous stirring. The phases are separated and the organic phase is washed twice with water, dried and evaporated to dryness. The residue is dissolved in dry EtOAc and the solution is again evaporated to dryness. The resulting diester free base which is obtained as an oil is again dissolved in 100 mL of dry ethyl acetate. To this solution is added a stream of dry HCl gas (ca. 6-8 mole equivalents) at 0°C under stirring in order to cleave the *t*-butyl ester. After stirring at 0°C for 2-3 hours, benazepril hydrochloride begins to crystallize. Stirring at room temperature is continued for an additional 4-5 hours. The suspension is cooled to 0°C and then filtered. The collected solid product is washed with ethyl acetate and finally dried in vacuum at 40°C to obtain benazepril hydrochloride of high purity.

## Claims

1. A compound which is a salt of a compound of the formula (II) 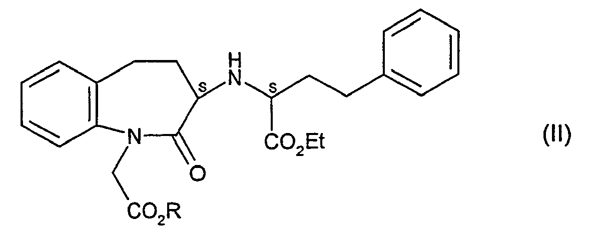 wherein
S denotes the (S)-configuration at the asymmetric carbon atoms; and
R represents a selectively removable protecting group;
said salt being a crystalline bis-dicarboxylic acid salt in which the molecular ratio of a compound of formula (II) to the dicarboxylic acid is 1:2.

2. A compound according to Claim 1, wherein R represents optionally substituted benzyl, optionally substituted benzhydryl, *t*-butyl, allyl, tetrahydrofuranyl or tetrahydropyranyl.

3. A compound according to Claim 1, wherein the dicarboxylic acid is of the formula (IV)
HOOC-X-COOH (IV)
wherein X is a direct bond, lower alkylene, arylene, or mono- or bis-hydroxy-lower alkylene, wherein lower alkylene represents straight-chain or branched alkylene of 1-7 carbon atoms.

4. A compound according to Claim 1, wherein the dicarboxylic acid is *D*- or *L*-tartaric acid, oxalic acid, optionally substituted (malonic acid, succinic acid or glutaric acid), *D*- or *L*-malic acid or *D*- or *L*-citramalic acid, or aspartic acid.

5. A compound according to Claim 1, wherein R is *t*-butyl and the dicarboxylic acid is *D-*or *L*-tartaric acid, oxalic acid or isopropylmalonic acid.

6. A process for the preparation of benazepril, the structural formula (I) of which is 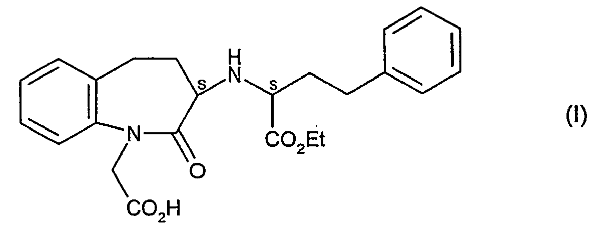 in which (S) denotes the (S)-configuration, or a pharmaceutically acceptable salt thereof, which comprises the following steps:
(a) purifying a diester of the formula (II) 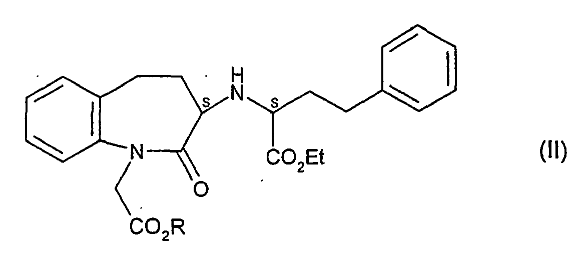 wherein
R represents a selectively removable protecting group; and
(S) denotes the (*S*)-configuration of the asymmetric carbon atoms,
by treatment with a dicarboxylic acid to obtain a crystalline *bis*-dicarboxylic acid salt thereof of high enantiomeric purity;
(b) converting said salt to the corresponding free base; and
(c) converting said diester free base to benazepril or a pharmaceutically acceptable salt thereof by selective removal of the R protecting group; and
(d) if required, converting the resulting benazepril free base to a pharmaceutically acceptable salt thereof.

7. A process according to Claim 6, wherein R is optionally substituted benzyl, optionally substituted benzhydryl, *t*-butyl, allyl, tetrahydrofuranyl or tetrahydropyranyl.

8. A process according to Claim 6, wherein the dicarboxylic acid is a compound of the formula (IV)
HOOC-X-COOH (IV)
wherein X represents a direct bond, lower alkylene, arylene or mono- or *bis*-hydroxy-lower alkylene, wherein lower alkylene represents straight-chain or branched alkylene of 1-7 carbon atoms.

9. A process according to Claim 6, wherein the dicarboxylic acid is oxalic acid, *D*- or *L*-tartaric acid, optionally substituted (malonic acid, glutaric acid or succinic acid), *D*- or *L*-malic acid, *D*- or *L*-citramalic acid or aspartic acid.

10. A process according to Claim 9, wherein the dicarboxylic acid is *D*- or *L*-tartaric acid, oxalic acid or isopropylmalonic acid.

11. A process according to Claim 6, wherein R is *t*-butyl.

12. A process according to Claim 11 for the preparation of benazepril hydrochloride, wherein Step (c) is carried out with HCl in an inert organic solvent.

13. A process according to Claim 6, wherein a compound of formula (II) is treated with 1 to 2 mole equivalents of the dicarboxylic acid.

## Patentansprüche

1. Verbindung, bei der es sich um ein Salz einer Verbindung der Formel (II) 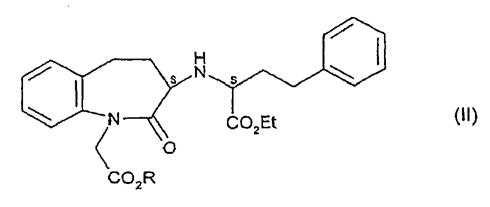 wobei
S die (S)-Konfiguration an den asymmetrischen Kohlenstoffatomen bedeutet; und
R eine selektiv entfernbare Schutzgruppe bedeutet; handelt, wobei dieses Salz ein kristallines Bisdicarbonsäuresalz ist, in dem das Molverhältnis einer Verbindung der Formel (II) zu der Dicarbonsäure 1:2 beträgt.

2. Verbindung nach Anspruch 1, wobei R gegebenenfalls substituiertes Benzyl, gegebenenfalls substituiertes Benzhydryl, *t*-Butyl, Allyl, Tetrahydrofuranyl oder Tetrahydropyranyl bedeutet.

3. Verbindung nach Anspruch 1, wobei die Dicarbonsäure der Formel (IV) entspricht,
HOOC-X-COOH (IV)
wobei X eine direkte Bindung, Niederalkylen, Arylen, oder Mono- oder Bis-hydroxy-Niederalkylen bedeutet, wobei Niederalkylen ein geradkettiges oder verzweigtes Alkylen mit 1-7 Kohlenstoffatomen bedeutet.

4. Verbindung nach Anspruch 1, wobei die Dicarbonsäure *D*- oder *L*-Weinsäure, Oxalsäure, gegebenenfalls substituierte (Malonsäure, Bernsteinsäure oder Glutarsäure), *D*- oder *L*-Äpfelsäure oder *D*- oder *L*-Citramalsäure, oder Asparaginsäure ist.

5. Verbindung nach Anspruch 1, wobei R *t*-Butyl ist und die Dicarbonsäure *D-* oder *L*-Weinsäure, Oxalsäure oder Isopropylmalonsäure ist.

6. Verfahren zur Herstellung von Benazepril, das die Strukturformel I 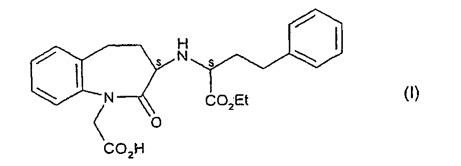 aufweist, in der (S) die (S)-Konfiguration bedeutet, oder eines seiner pharmazeutisch unbedenklichen Salze, wobei das Verfahren die folgenden Schritte umfaßt:
(a) Aufreinigen eines Diesters der Formel (II) 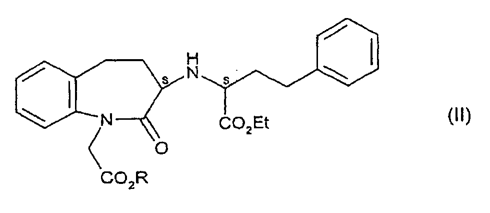 in der
R eine selektiv entfernbare Schutzgruppe bedeutet; und
(S) die (S)-Konfiguration der asymmetrischen Kohlenstoffatome bedeutet;
durch Behandeln mit einer Dicarbonsäure, wodurch man zu einem kristallinen Bis-dicarbonsäuresalz davon
gelangt, wobei dieses Salz hoch enantiomerenrein ist;
(b) Umwandeln dieses Salzes in die entsprechende freie Base; und
(c) Umwandeln der freien Base des Diesters in Benazepril oder eines seiner pharmazeutisch unbedenklichen Salze durch selektive Entfernung der R-Schutzgruppe; und
(d) falls erforderlich, Umwandeln der erhaltenen freien Benazepril-Base in eines ihrer pharmazeutisch unbedenklichen Salze.

7. Verfahren nach Anspruch 6, wobei R gegebenenfalls substituiertes Benzyl, gegebenenfalls substituiertes Benzhydryl, *t*-Butyl, Allyl, Tetrahydrofuranyl oder Tetrahydropyranyl ist.

8. Verfahren nach Anspruch 6, wobei die Dicarbonsäure eine Verbindung der Formel (IV) ist,
HOOC-X-COOH (IV)
wobei X eine direkte Bindung, Niederalkylen, Arylen oder Mono- oder Bis-hydroxy-Niederalkylen bedeutet, wobei Niederalkylen geradkettiges oder verzweigtes Alkylen mit 1-7 Kohlenstoffatomen bedeutet.

9. Verfahren nach Anspruch 6, wobei die Dicarbonsäure, Oxalsäure, *D*- oder *L*-Weinsäure, gegebenenfalls substituierte (Malonsäure, Glutarsäure oder Bernsteinsäure), *D*- oder *L*-Äpfelsäure oder *D*- oder *L*-Citramalsäure, oder Asparaginsäure ist.

10. Verfahren nach Anspruch 9, wobei die Dicarbonsäure *D*- oder *L*-Weinsäure, Oxalsäure oder Isopropylmalonsäure ist.

11. Verfahren nach Anspruch 6, wobei R *t*-Butyl ist.

12. Verfahren nach Anspruch 11 zur Herstellung von Benazepril-hydrochlorid, wobei Schritt (c) mit HCl in einem inerten organischen Lösungsmittel durchgeführt wird.

13. Verfahren nach Anspruch 6, wobei eine Verbindung der Formel (II) mit 1 bis 2 Moläquivalenten der Dicarbonsäure behandelt wird.

## Revendications

1. Composé qui est un sel d'un composé de formule (II) : 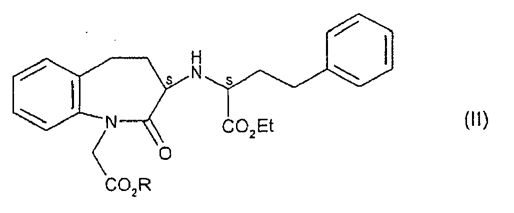 dans laquelle
(S) désigne la configuration (S) des atomes de carbone asymétriques ; et
R désigne un groupe protecteur amovible de façon sélective ;
ledit sel étant un sel d'acide bis-dicarboxylique dans lequel le rapport moléculaire entre le composé de formule (II) et l'acide dicarboxylique est de 1:2.

2. Composé selon la revendication 1, dans lequel R désigne un benzyle éventuellement substitué, un benzhydryle éventuellement substitué, un t-butyle, un allyle, un tétrahydrofuranyle ou un tétrahydropyranyle.

3. Composé selon la revendication 1, dans lequel l'acide dicarboxylique est représenté par la formule (IV) :
HOOC-X-COOH (IV)
dans laquelle X désigne une liaison directe, un alkylène inférieur, un arylène ou un alkylène mono- ou bis-hydroxy inférieur, le terme "alkylène inférieur" désignant un alkylène à chaîne droite ou ramifié ayant 1 à 7 atomes de carbone.

4. Composé selon la revendication 1, dans lequel l'acide dicarboxylique est l'acide D- ou L-tartrique, l'acide oxalique, les acides malonique, succinique ou glutarique éventuellement substitués, l'acide D- ou L-malique, l'acide D- ou L-citramalique ou l'acide aspartique.

5. Composé selon la revendication 1, dans lequel R est un t-butyle et l'acide dicarboxlyique est l'acide D- ou L-tartrique, l'acide oxalique ou l'acide isopropylmalonique.

6. Procédé de préparation de bénazépril, dont la formule structurale (I) est : 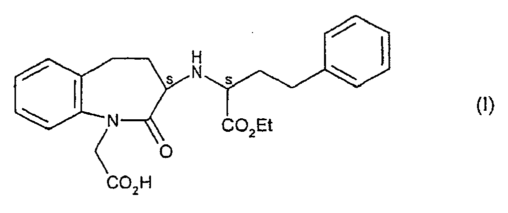 dans laquelle (S) désigne la configuration (S), ou d'un sel pharmaceutiquement acceptable de celui-ci, qui comprend les étapes suivantes :
(a) purification d'un diester de formule (II) : 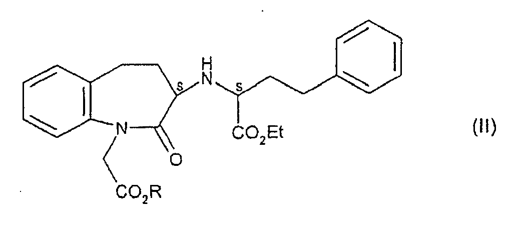 dans laquelle
R désigne un groupe protecteur amovible de façon sélective ; et
(S) désigne la configuration (S) des atomes de carbone asymétriques ;
par traitement avec un acide dicarboxylique pour obtenir un sel d'acide bis-dicarboxylique cristallin de celui-ci de pureté énantiomérique élevée ;
(b) conversion dudit sel en base libre correspondante ; et
(c) conversion de ladite base libre de diester en bénazépril ou en un sel pharmaceutiquement acceptable de celui-ci par élimination sélective du groupe protecteur R ; et
(d) le cas échéant, conversion de la base libre de bénazépril résultante en un sel pharmaceutiquement acceptable de celui-ci.

7. Procédé selon la revendication 6, dans lequel dans lequel R désigne un benzyle éventuellement substitué, un benzhydryle éventuellement substitué, un t-butyle, un allyle, un tétrahydrofuranyle ou un tétrahydropyranyle.

8. Procédé selon la revendication 6, dans lequel l'acide dicarboxylique est un composé de formule (IV) :
HOOC-X-COOH (IV)
dans laquelle X désigne une liaison directe, un alkylène inférieur, un arylène ou un alkylène mono- ou bis-hydroxy inférieur, le terme "alkylène inférieur" désignant un alkylène à chaîne droite ou ramifié ayant 1 à 7 atomes de carbone.

9. Procédé selon la revendication 6, dans lequel l'acide dicarboxylique est l'acide oxalique, l'acide D-ou L-tartrique, les acides malonique, glutarique ou succinique éventuellement substitués, l'acide D- ou L-malique, l'acide D- ou L-citramalique ou l'acide aspartique.

10. Procédé selon la revendication 9, dans lequel l'acide dicarboxlyique est l'acide D- ou L-tartrique, l'acide oxalique ou l'acide isopropylmalonique.

11. Procédé selon la revendication 6, dans lequel R est un t-butyle.

12. Procédé selon la revendication 11 de préparation de chlorure de bénazépril, dans lequel l'étape (c) est effectuée avec de l'HCl dans un solvant organique inerte.

13. Procédé selon la revendication 6, dans lequel un composé de formule (II) est traité avec 1 à 2 équivalents molaires de l'acide dicarboxylique.
